# EUROPEAN PATENT SPECIFICATION

(11) **EP 2 473 503 B1**
(45) Date of publication and mention of the grant of the patent: **23.07.2014**
(21) Application number: 10752548.7
(22) Date of filing: 02.09.2010
(51) Int. Cl.: C07D 401/14, C07D 403/14, A61K 31/505, A61P 31/12

(54) **BIS-BENZIMIDAZOLE DERIVATIVES**
BIS-BENZIMIDAZOLDERIVATIVE
DÉRIVÉS DE BIS-BENZIMIDAZOLE

(30) Priority: 03.09.2009 EP 09169386
(43) Date of publication of application: 11.07.2012
(73) Proprietor: Janssen R&D Ireland, Eastgate, Little Island, County Cork (IE)
(72) Inventor: VANDYCK, Koen, B-3583 Paal-Beringen (BE); MC GOWAN, David, B-1150 Brussel (BE); RABOISSON, Pierre Jean-Marie Bernard, B-1331 Rosieres (BE)
(74) Representative: van Wanrooij, Eva
(86) International application number: PCT/EP2010/062911
(87) International publication number: WO 2011/026920

(56) References cited:
- WO-A1-2006/133326
- WO-A1-2010/091413

## Description

### Technical Field

This invention relates to bis-benzimidazole derivatives, which are inhibitors of the hepatitis C virus (HCV), their synthesis and their use, alone or in combination with other HCV inhibitors in the treatment or prophylaxis of HCV.

### Background Art

HCV is a single stranded, positive-sense RNA virus belonging to the *Flaviviridae* family of viruses in the hepacivirus genus. The viral genome translates into a single open reading frame that encodes for multiple structural and nonstructural proteins.

Following the initial acute infection, a majority of infected individuals develop chronic hepatitis because HCV replicates preferentially in hepatocytes but is not directly cytopathic. In particular, the lack of a vigorous T-lymphocyte response and the high propensity of the virus to mutate appear to promote a high rate of chronic infection. Chronic hepatitis can progress to liver fibrosis, leading to cirrhosis, end-stage liver disease, and HCC (hepatocellular carcinoma), making it the leading cause of liver transplantations.

There are six major HCV genotypes and more than 50 subtypes, which are differently distributed geographically. HCV genotype 1 is the predominant genotype in Europe and in the US. The extensive genetic heterogeneity of HCV has important diagnostic and clinical implications, perhaps explaining difficulties in vaccine development and the lack of response to current therapy.

Transmission of HCV can occur through contact with contaminated blood or blood products, for example following blood transfusion or intravenous drug use. The introduction of diagnostic tests used in blood screening has led to a downward trend in post-transfusion HCV incidence. However, given the slow progression to the end-stage liver disease, the existing infections will continue to present a serious medical and economic burden for decades.

Current HCV therapies are based on (pegylated) interferon-alpha (IFN-α) in combination with ribavirin. This combination therapy yields a sustained virologic response in 40% of patients infected by genotype 1 HCV and about 80% of those infected by genotypes 2 and 3. Beside the limited efficacy on HCV genotype 1, this combination therapy has significant side effects including influenza-like symptoms, hematologic abnormalities, and neuropsychiatric symptoms. Hence there is a need for more effective, convenient and better-tolerated treatments.

Experience with HIV drugs, in particular with HIV protease inhibitors, has taught that sub-optimal pharmacokinetics and complex dosing regimens quickly result in inadvertent compliance failures. This in turn means that the 24 hour trough concentration (minimum plasma concentration) for the respective drugs in an HIV regime frequently falls below the IC₉₀ or ED₉₀ threshold for large parts of the day. It is considered that a 24 hour trough level of at least the IC₅₀, and more realistically, the IC₉₀ or ED₉₀, is essential to slow down the development of drug escape mutants. Achieving the necessary pharmacokinetics and drug metabolism to allow such trough levels provides a stringent challenge to drug design.

The NS5A protein of HCV is located downstream of the NS4B protein and upstream of the NS5B protein. Upon posttranslational cleavage by the viral serine protease NS3/4A, the NS5A matures into a zinc containing, three-domain phosphoprotein that either exists as a hypophosphorylated (56-kDa, p56) or hyperphosphorylated species (58-kDa, p58). NS5A of HCV is implicated in multiple aspects of the viral lifecycle including viral replication and infectious particle assembly as well as modulation of the environment of its host cell. Although no enzymatic function has been ascribed to the protein it is reported to interact with numerous viral and cellular factors.

A number of patents and patent applications disclose compounds with HCV inhibitory activity, in particular targeting NS5A. WO2006/133326 discloses stilbene derivatives while WO 2008/021927 and WO 2008/021928 disclose biphenyl derivatives having NS5A HCV inhibitory activity. WO 2008/048589 discloses 4-(phenylethynyl)-1*H-*pyrazole derivatives and their antiviral use. WO 2008/070447 discloses a broad range of HCV inhibiting compounds including compounds with a benzimidazole moiety. WO-2010/017401, WO-2010/065681 and WO-2010/091413 disclose bis-benzimidazole inhibitors of HCV NS5A.

There is a need for HCV inhibitors that may overcome the disadvantages of current HCV therapy such as side effects, limited efficacy, the emerging of resistance, and compliance failures, as well as improve the sustained viral load response.

The present invention concerns a group of HCV inhibiting bis-benzimidazole derivatives with useful properties regarding one or more of the following parameters: antiviral efficacy, favorable profile of resistance development, reduced or lack of toxicity and genotoxicity, favorable pharmacokinetics and pharmacodynamics, ease of formulation and administration and limited or lack of drug-drug interactions with other drug substances, in particular other anti-HCV agents.

Compounds of the invention may also be attractive due to the fact that they lack activity against other viruses, in particular against HIV. HIV infected patients often suffer from co-infections such as HCV. Treatment of such patients with an HCV inhibitor that also inhibits HIV may lead to the emergence of resistant HIV strains.

### Description of the Invention

In one aspect, the present invention provides compounds, which can be represented by the formula I: 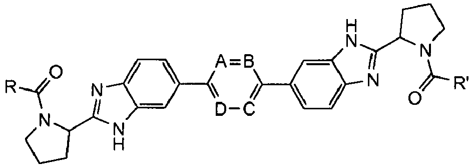 including any possible stereoisomers thereof, wherein:
A, B, C and D are independently -CH= or -N=, provided one or two of A, B, C and D is -N= and the remainder is -CH=;
R and R' are independently selected from -CR₁R₂R₃, aryl optionally substituted with 1 or 2 substituents selected from halo and methyl, or heteroC₃₋₆cycloalkyl, wherein
   R₁ is selected from C₁₋₄alkyl optionally substituted with methoxy or dimethylamino; phenyl optionally substituted with 1, 2 or 3 substituents independently selected from halo, C₁₋₄alkoxy, trifluoromethoxy or 2 substituents on adjacent ring atoms form a 1,3-dioxolane group; benzyl optionally substituted with halo or methoxy; C₃₋₆cycloalkyl; heteroaryl; heteroC₃₋₆cycloalkyl and heteroarylmethyl;
R₂ is selected from hydrogen, hydroxyl, amino, mono- or di-C₁₋₄alkylamino, C₁₋₄alkylcarbonylamino, C₁₋₄alkyloxycarbonylamino, C₁₋₄alkylaminocarbonyl-amino, piperidin-1-yl and imidazol-1-yl; and
R₃ is hydrogen, or R₁ and R₃ together form an oxo or a cyclopropyl group;
or a pharmaceutically acceptable salt and/or solvate thereof.

The invention particularly relates to a compound of formula I 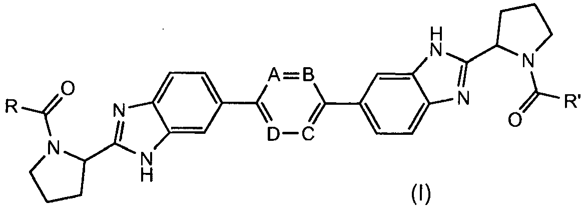 or a stereoisomer thereof, wherein:
A, B, C and D are independently -CH= or -N=, provided that one or two of A, B, C and D is -N= and the remainder is -CH=;
R and R' are independently selected from -CR₁R₂R₃, aryl optionally substituted with 1 or 2 substituents selected from halo and methyl, and heteroC₄₋₇cycloalkyl, wherein
   R₁ is selected from the group consisting of C₁₋₄alkyl optionally substituted with methoxy or dimethylamino; phenyl optionally substituted with 1, 2 or 3 substituents independently selected from halo, C₁₋₄alkoxy, trifluoromethoxy or 2 substituents on adjacent ring atoms form a 1,3-dioxolane group; benzyl optionally substituted with halo or methoxy; C₃₋₆cycloalkyl; heteroaryl; heteroC₄₋₇cyclo-alkyl; and heteroarylmethyl;
   R₂ is selected from hydrogen, hydroxyl, amino, mono- and di-C₁₋₄alkylamino, C₁₋₄alkylcarbonylamino, C₁₋₄alkyloxycarbonylamino, C₁₋₄alkylamino-carbonylamino, piperidin-1-yl and imidazol-1-yl; and
   R₃ is hydrogen, or R₁ and R₃ together form a cyclopropyl group; or R₂ and R₃ together form oxo;
or a pharmaceutically acceptable salt or a solvate thereof, provided that 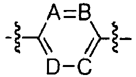 is other than pyrazine or pyridine when R and R' both are (S)-1-methoxycarbonylamino-2-methyl-propan-1-yl; and that R² is other than methoxycarbonylamino when R¹ is phenyl and R³ is hydrogen.

In a further aspect, the invention concerns the use of compounds of formula I, or subgroups thereof, as specified herein, for inhibiting HCV. Alternatively, there is provided the use of said compounds for the manufacture of a medicament for inhibiting HCV.

Embodiments of the present invention concerns compounds of formula (I), or any subgroup thereof as defined herein, wherein one or more of the definitions for R, R', R₁, R₂ and R₃ as specified herein, apply.

Subgroups of compounds of formula I are those compounds of formula I, or subgroups of compounds of formula I, as defined herein, wherein R and R' are independently -CR₁R₂R₃ or aryl wherein aryl is 5-membered heteroaryl; in particular, wherein R and R' are independently is -CR₁R₂R₃; more in particular, wherein R and R' are -CR₁R₂R₃ and are the same.

Subgroups of compounds of formula I are those compounds of formula I, or subgroups of compounds of formula I, as defined herein, wherein R₂ is hydroxyl, amino, mono- or di-C₁₋₄alkylamino, C₁₋₄alkylcarbonylamino, C₁₋₄alkyloxycarbonylamino; in particular, R₂ is C₁₋₄alkylcarbonylamino or C₁₋₄alkyloxycarbonylamino.

Subgroups of compounds of formula I are those compounds of formula I, or subgroups of compounds of formula I, as defined herein, wherein R₁ is selected from C₁₋₄alkyl; phenyl optionally substituted with 1 or 2 substituents independently selected from halo, methyl, methoxy or 2 substituents on adjacent ring atoms form a 1,3-dioxolane group; and heteroaryl. In particular, R₁ is selected from branched C₃₋₄alkyl; phenyl optionally substituted with 1 substituents selected from halo and methyl; and heteroaryl. More in particular, R₁ is selected from branched C₃₋₄alkyl; phenyl optionally substituted with 1 substituent selected from halo.

Subgroups of compounds of formula I are those compounds of formula I, or subgroups of compounds of formula I, as defined herein, wherein 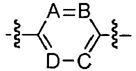 is selected from pyrimidine (i.e. A and D are -N=), pyridazine (i.e. A and B are -N=), pyrazine (i.e. A and C are -N=) and pyridine (i.e. A is =N=); in particular, 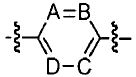 is pyrimidine.

In a further aspect, the invention provides a compound of formula I or a pharmaceutically acceptable salt, hydrate, or solvate thereof, for use in the treatment or prophylaxis (or the manufacture of a medicament for the treatment or prophylaxis) of HCV infection. Representative HCV genotypes in the context of treatment or prophylaxis in accordance with the invention include genotype 1b (prevalent in Europe) or 1a (prevalent in North America). The invention also provides a method for the treatment or prophylaxis of HCV infection, in particular of the genotype 1a or 1b.

In a first embodiment R and R' are independently selected from -CR₁R₂R₃.

In a second embodiment R and R' are the same.

In a third embodiment R₂ is C₁₋₄alkylcarbonylamino or C₁₋₄alkyloxycarbonylamino.

In a fourth embodiment R₁ is selected from branched C₃₋₄alkyl; phenyl optionally substituted with 1 substituent selected from halo and methyl; and heteroaryl.

In a fifth embodiment R₁ is selected from C₁₋₄alkyl optionally substituted with methoxy; phenyl optionally substituted with halo, and C₃₋₆cycloalkyl.

In a sixth embodiment the compound is of formula Ia.

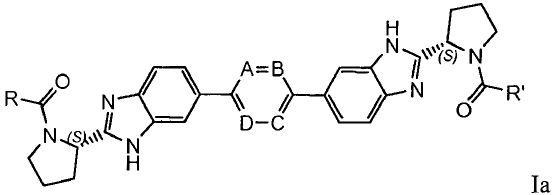

In a seventh embodiment, 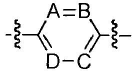 is pyrimidine or pyridazine.

Pure stereoisomeric forms of the compounds and intermediates as mentioned herein are defined as isomers substantially free of other enantiomeric or diastereomeric forms of the same basic molecular structure of said compounds or intermediates. In particular, the term "stereoisomerically pure" concerns compounds or intermediates having a stereoisomeric excess of at least 80% (i.e. minimum 90% of one isomer and maximum 10% of the other possible isomers) up to a stereoisomeric excess of 100% (i.e. 100% of one isomer and none of the other), more in particular, compounds or intermediates having a stereoisomeric excess of 90% up to 100%, even more in particular having a stereoisomeric excess of 94% up to 100% and most in particular having a stereoisomeric excess of 97% up to 100%. The terms "enantiomerically pure" and "diastereomerically pure" should be understood in a similar way, but then having regard to the enantiomeric excess, and the diastereomeric excess, respectively, of the mixture in question.

Pure stereoisomeric forms or stereoisomers of the compounds and intermediates of this invention may be obtained by the application of art-known procedures. For instance, enantiomers may be separated from each other by the selective crystallization of their diastereomeric salts with optically active acids or bases. Examples thereof are tartaric acid, dibenzoyltartaric acid, ditoluoyltartaric acid and camphorsulfonic acid. Alternatively, enantiomers may be separated by chromatographic techniques using chiral stationary phases. Said pure stereochemically isomeric forms may also be derived from the corresponding pure stereoisomeric forms of the appropriate starting materials, provided that the reaction occurs stereospecifically. Preferably, if a specific stereoisomer is desired, said compound is synthesized by stereospecific methods of preparation. These methods will advantageously employ enantiomerically pure starting materials.

The diastereomeric racemates of the compounds of formula I can be obtained separately by conventional methods. Appropriate physical separation methods that may advantageously be employed are, for example, selective crystallization and chromatography, e.g. column chromatography or supercritical fluid chromatography.

The compounds of formula I have several centers of chirality. Of interest are the stereogenic centers of the pyrrolidine ring at the 2-carbon atom. The configuration at this position may be that corresponding to L-proline, i.e. 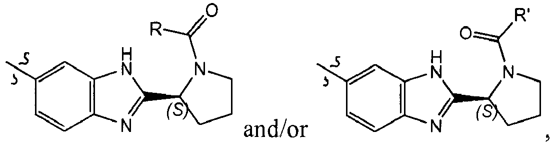 or that corresponding to D-proline, i.e.

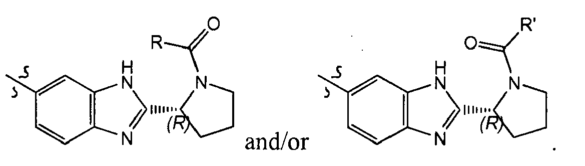

Of particular interest are compounds of formula I or subgroups thereof as defined herein, that are according to formula Ia.

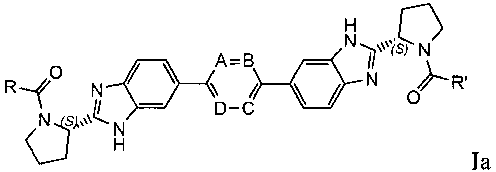

Also of interest is the configuration of the group -CR₁R₂R₃: when R₁ is selected from C₁₋₄alkyl optionally substituted with methoxy, hydroxyl or dimethylamino; C₃₋₆cycloalkyl; and tetrahydropyranyl, then the S-configuration is preferred; when R₁ is selected from phenyl optionally substituted with 1, 2 or 3 substituents independently selected from halo, C₁₋₄alkoxy, trifluoromethoxy or 2 substituents on adjacent ring atoms form a 1,3-dioxolane group; and heteroaryl; then the R-configuration is preferred.

The pharmaceutically acceptable addition salts comprise the therapeutically active non-toxic acid and base addition salt forms of the compounds of formula (I) or subgroups thereof. Of interest are the free, i.e. non-salt forms of the compounds of formula I, or of any subgroup of compounds of formula I specified herein.

The pharmaceutically acceptable acid addition salts can conveniently be obtained by treating the base form with such appropriate acid. Appropriate acids comprise, for example, inorganic acids such as hydrohalic acids, e.g. hydrochloric or hydrobromic acid, sulfuric, nitric, phosphoric and the like acids; or organic acids such as, for example, acetic, propionic, hydroxyacetic, lactic, pyruvic, oxalic (i.e. ethanedioic), malonic, succinic (i.e. butanedioic acid), maleic, fumaric, malic (i.e. hydroxyl-butanedioic acid), tartaric, citric, methanesulfonic, ethanesulfonic, benzenesulfonic, *p*-toluenesulfonic, cyclamic, salicylic, *p*-aminosalicylic, pamoic and the like acids. Conversely said salt forms can be converted by treatment with an appropriate base into the free base form.

The compounds of formula (I) containing an acidic proton may also be converted into their base addition salts, in particular metal or amine addition salt forms, by treatment with appropriate organic and inorganic bases. Appropriate base salt forms comprise, for example, the ammonium salts, the alkali and earth alkaline metal salts, e.g. the lithium, sodium, potassium, magnesium, calcium salts and the like, salts with organic bases, e.g. the benzathine, *N*-methyl-D-glucamine, hydrabamine salts, and salts with amino acids such as, for example, arginine, lysine and the like.

The term "solvates" covers any pharmaceutically acceptable solvates that the compounds of formula I as well as the salts thereof, are able to form. Such solvates are for example hydrates, alcoholates, e.g. ethanolates, propanolates, and the like.

Some of the compounds of formula I may also exist in tautomeric forms. For example, tautomeric forms of amide (-C(=O)-NH-) groups are iminoalcohols (-C(OH)=N-). Tautomeric forms, although not explicitly indicated in the structural formulae represented herein, are intended to be included within the scope of the present invention.

As used herein, "C₁₋₄alkyl" as a group or part of a group defines saturated straight or branched chain hydrocarbon groups having from 1 to 4 carbon atoms such as for example methyl, ethyl, 1-propyl, 2-propyl, 1-butyl, 2-butyl, 2-methyl-1-propyl, 2-methyl-2-propyl. For the purpose of the present invention, of interest amongst C₁₋₄alkyl is C₃₋₄alkyl, i.e. straight or branched chain hydrocarbon groups having 3 or 4 carbon atoms such as 1-propyl, 2-propyl, 1-butyl, 2-butyl, 2-methyl-1-propyl, 2-methyl-2-propyl. Of particular interest may be branched C₃₋₄alkyl such as 2-propyl, 2-butyl, 2-methyl-1-propyl, 2-methyl-2-propyl.

The term "C₃₋₆cycloalkyl" as a group or part thereof, defines saturated cyclic hydrocarbon groups having from 3 to 6 carbon atoms that together form a cyclic structure. Examples of C₃₋₆cycloalkyl include cyclopropyl, cyclobutyl, cyclopentyl and cyclohexyl.

"C₁₋₄alkoxy" as a group or part of a group means a group of formula -O-C₁₋₄alkyl wherein C₁₋₄alkyl is as defined above. Examples of C₁₋₄alkoxy are methoxy, ethoxy, n-propoxy, or isopropoxy.

The term "halo" is generic to fluoro, chloro, bromo and iodo.

As used herein, the term "(=O)" or "oxo" forms a carbonyl moiety when attached to a carbon atom. It should be noted that an atom can only be substituted with an oxo group when the valency of that atom so permits.

As used herein for the purpose of defining "aryl" as a group or part thereof means an aromatic ring structure optionally comprising one or two heteroatoms selected from N, O and S, in particular from N and O. Said aromatic ring structure may have 5 or 6 ring atoms.

As used herein, the prefix "hetero-" means that the group comprises or includes at least 1 heteroatom selected from N, O and S, in particular N and O. For example, the term "heteroaryl" means an aromatic ring structure as defined for the term "aryl" comprising at least 1 heteroatom selected from N, O and S, in particular from N and O. Alternatively, the term "heteroC₄₋₇cycloalkyl" means a saturated cyclic hydrocarbon wherein at least 1 carbon atom is replaced by a heteroatom selected from N, O and S, in particular from N and O. Examples of heteroC₄₋₇Cycloalkyl include tetrahydro-2H-pyranyl, piperidinyl, tetrahydrofuranyl, and pyrrolidinyl.

Where the position of a group on a molecular moiety is not specified (for example a substituent on phenyl) or is represented by a floating bond, such group may be positioned on any atom of such a moiety, as long as the resulting structure is chemically stable. When any variable is present more than once in the molecule, each definition is independent.

Whenever used herein, the term "compounds of formula I", or "the present compounds" or similar terms, it is meant to include the compounds of formula I, including the possible stereoisomeric forms, and the pharmaceutically acceptable salts and solvates thereof.

### General synthetic methods

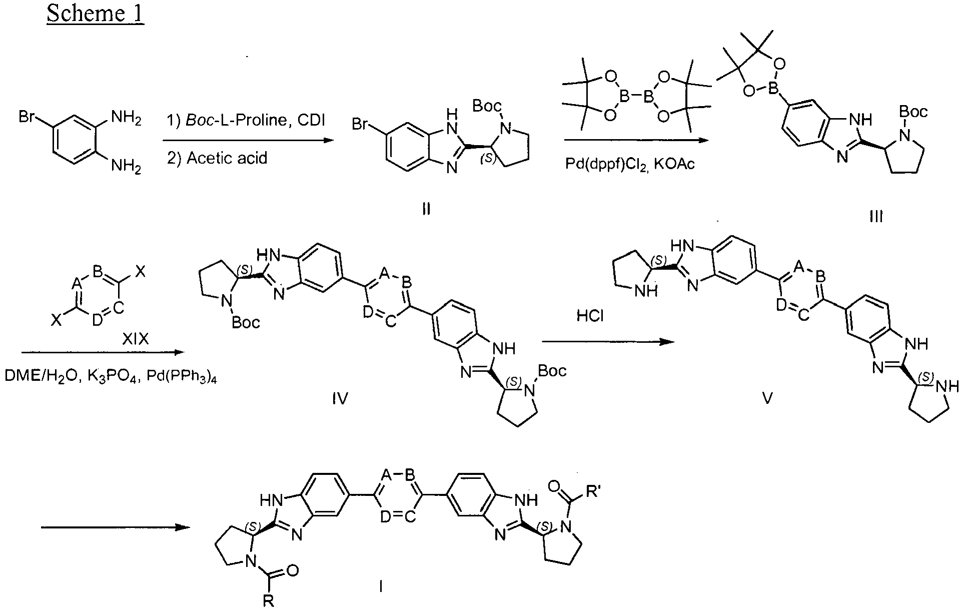

Compounds of formula I wherein R and R' are the same, can be obtained using the synthetic pathway illustrated in the scheme 1 above. Coupling of Boc-L-Proline with 4-bromobenzene-1,2-diamine, in the presence of, for example, CDI, followed by cyclisation by heating in acetic acid, results in benzimidazole derivative **II.** This compound can be converted to boronic ester **III** under Pd catalyzed conditions in the presence of bis(pinacolato)diboron. Subsequently, boronic ester **III** is converted to compound **IV,** by coupling under Suzuki-Miyaura conditions with a six-membered heterocylic bis halogenide of formula XIX, wherein A, B, C and D have the meaning as defined herein for compounds of formula I or subgroups thereof, and X is a halogen; in particular selected from iodo, chloro and bromo; more in particular X is iodo.

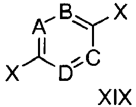

Compound **V** is obtained after removal of the Boc protecting group of the proline nitrogen under acidic conditions, for example using HCl in isopropanol. The resulting compound **V** may then be converted to a compound of formula I by acylation with the appropriate acid of formula R-C(=O)-OH or R'-C(=O)-OH wherein R and R' have the meanings as defined for the compounds of formula I or any subgroup thereof.
Said acylation may be performed by reacting the starting materials in the presence of a coupling agent or by converting the carboxyl functionality into an active form such as an active ester, mixed anhydride or a carboxyl acid chloride or bromide. General descriptions of such coupling reactions and the reagents used therein can be found in general textbooks on peptide chemistry, for example, M. Bodanszky, "Peptide Chemistry", 2nd rev. ed., Springer-Verlag, Berlin, Germany, (1993).

Examples of coupling reactions for amino-group acylation or amide bond formation include the azide method, mixed carbonic-carboxylic acid anhydride (isobutyl chloro-formate) method, the carbodiimide (dicyclohexylcarbodiimide, diisopropylcarbodiimide, or water-soluble carbodiimide such as *N*-ethyl-*N'*-[3-(dimethylamino)-propyl]carbodiimide) method, the active ester method (e.g. *p*-nitrophenyl, *p*-chloro-phenyl, trichlorophenyl, pentachloro-phenyl, pentafluorophenyl, *N*-hydroxysuccinic imido and the like esters), the Woodward reagent K-method, the 1,1-carbonyl-diimidazole (CDI or *N*,*N*'-carbonyl-diimidazole) method, the phosphorus reagents or oxidation-reduction methods. Some of these methods can be enhanced by adding suitable catalysts, e.g. in the carbodiimide method by adding 1-hydroxybenzotriazole, or 4-DMAP. Further coupling agents are (benzotriazol-1-yloxy)-*tris*-(dimethylamino) phosphonium hexafluorophosphate, either by itself or in the presence of 1-hydroxy-benzotriazole or 4-DMAP; or 2-(1*H*-benzotriazol-1-yl)-*N*,*N*,*N'*,*N'*-tetramethyluronium tetrafluoroborate (TBTU), or O-(7-azabenzotriazol-1-yl)-*N*,*N*,*N'*,*N*'-tetramethyluronium hexafluorophosphate (HATU). These coupling reactions can be performed in either solution (liquid phase) or solid phase. For the purpose of the present invention, a preferred method for acylation is performed employing HATU.

The coupling reactions preferably are conducted in an inert solvent, such as halogenated hydrocarbons, e.g. dichloromethane (DCM), chloroform, dipolar aprotic solvents such as acetonitrile, dimethylformamide, dimethylacetamide, DMSO, HMPT, ethers such as tetrahydrofuran (THF).

In many instances the coupling reactions are done in the presence of a suitable base such as a tertiary amine, e.g. triethylamine, diisopropylethylamine (DIPEA), N-methyl-morpholine, N-methylpyrrolidine, 4-DMAP or 1,8-diazabicyclo[5.4.0]undec-7-ene (DBU). The reaction temperature may range between 0°C and 50°C and the reaction time may range between 15 min and 24 h.

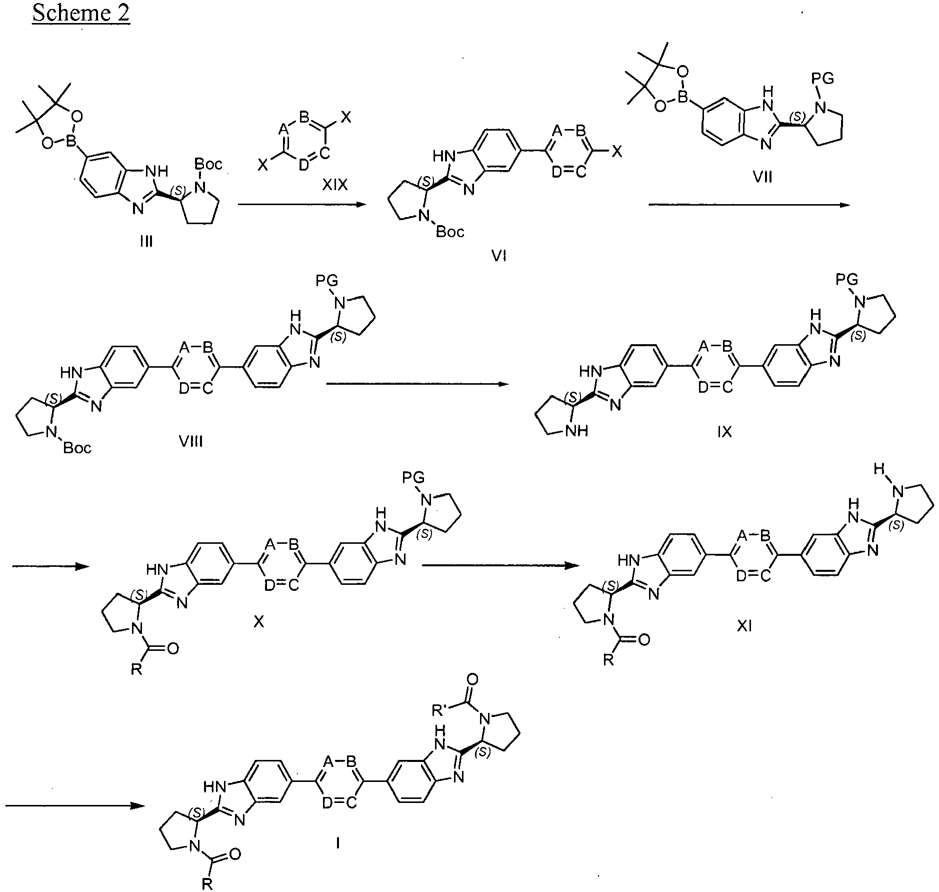

Alternatively, compounds of formula **I** wherein R and R' are not the same, may be obtained by using the synthetic pathway as illustrated by scheme 2. Using standard Suzuki-Miyaura conditions, boronic ester **III** and six-membered heterocylic bis halogenide **XIX** can be coupled, under conditions comparable to those used in the conversion **of III** to **IV** (scheme 1), except that the ratio of six-membered heterocylic bis halogenide **XIX** to boronic ester **III** is around 1 to 1, more likely higher, obtaining mono halogenide **VI. VI** can then be coupled with boronic ester **VII.** It should be understood that the amino protecting group PG occupying the pyrrolidine nitrogen in boronic ester **VII** should be selected so that it can be removed under conditions that do not affect a Boc-group or do not affect an R-C(=O)- group on an alternative nitrogen in the molecule. It should also be understood that PG may as well be the R'-C(=O)- group of the final compound of formula I being synthesized. Coupling of **VI** and **VII** may again be performed by using standard Suzuki-Miyaura conditions, and results in compound **VIII.** Compound **VIII** can then selectively be deprotected to compound **IX** by using conditions appropriate to remove the Boc protecting group. For example, in case PG is benzyloxycarbonyl or benzyl, the Boc protecting group may by selectively removed under standard Boc-deprotection conditions, i.e. acid treatment.
Other suitable protection groups PG and concomittant selective deprotection conditions can be sourced from Greene's "Protective groups in organic synthesis" by Peter G. M. Wuts, Fourth Edition, Chapter 7: 'Protection for the Amino group'.
Compound **IX** is subsequently acylated with the appropriate acid of formula R-C(=O)-OH wherein R has the meanings of R as defined for the compounds of formula I or any subgroup thereof. Compound **X** is obtained.

For compound **X**, in case PG represents -(C=O)-R', compound **X** equals compound **I**. In case PG represents an amino protecting group, PG can be removed under suitable conditions, allowing compatibility with -(C=O)-R, for example hydrogenation if PG is benzyl or benzyloxycarbonyl or basic conditions like diethylamine in case PG is fluorenylmethyloxycarbonyl, resulting in compound **XI.** Other selective deprotections can be sourced from Greene's Protective groups in organic synthesis" by Peter G. M. Wuts, Fourth Edition, Chapter 7: 'Protection for the Amino group'.
Compounds **XI** can be transformed in compound **I**, by acylation similar to the conversion of **IX** to **X** and as described in detail for the conversion of V to I under scheme 1.

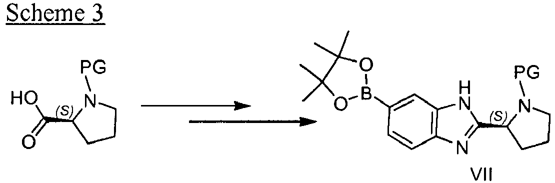

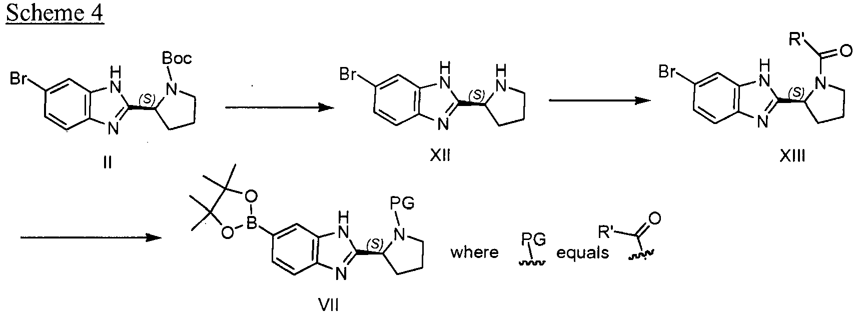

The boronic ester **VII** can be obtained by at least two different routes as illustrated in schemes 3 and 4. In case PG equals a protecting groups like for example benzyloxycarbonyl, fluorenylmethyloxycarbonyl, benzyl, or a other suitable protection group PG as described in Greene's Protective groups in organic synthesis" by Peter G. M. Wuts, Fourth Edition, Chapter 7: 'Protection for the Amino group', the compound can be synthesized by methods used for the synthesis of intermediate compound **II** and boronic ester **III,** starting from the correspondingly protected proline derivative as shown in scheme 3. In case PG equals -(C=O)-R', the compound can be made from intermediate **II,** as shown in scheme 4, by deprotection of the proline nitrogen under acidic conditions, such as treatment with HCl in for example iPrOH or trifluoroacetic acid resulting in compound **XII,** followed by coupling under standard acylation conditions, like the use of HATU in presence of a base like Hunig's base. Next, the obtained bromide **XIII** can be transformed in boronic acid **VII** (where PG equals -(C=O)-R'), like for example the transformation of **II** to **III.**

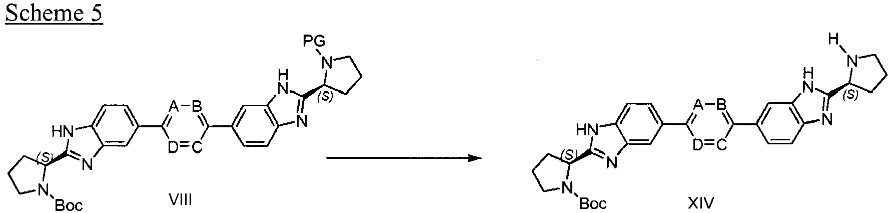

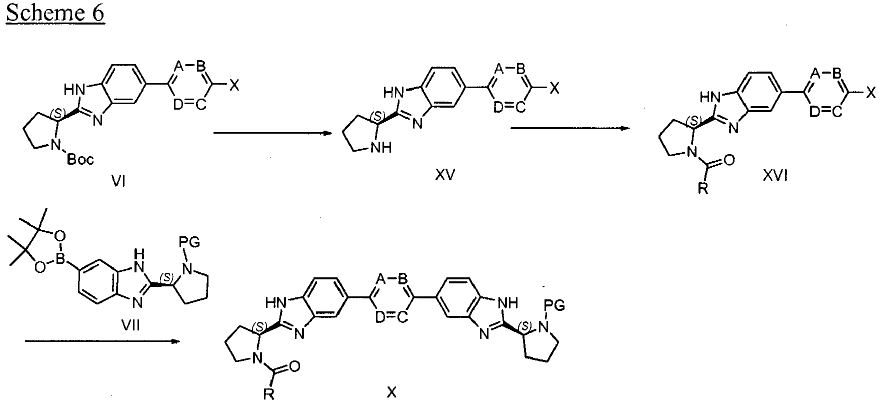

Alternatively, as shown in scheme 5, compound **VIII** can be deprotected under suitable conditions, allowing compatibility with the tBu-oxy carbonyl protection, for example hydrogenation if PG is benzyl or benzyloxycarbonyl or basic conditions like diethylamine in case PG is fluorenylmethyloxycarbonyl, resulting in compound **XIV.** Other selective deprotections can be sourced from Greene's "Protective groups in organic synthesis" by Peter G. M. Wuts, Fourth Edition, Chapter 7: 'Protection for the Amino group'. In this case, compound **XIV** equals compound **IX,** with PG being tert-butoxycarbonyl (Boc). In this case, deprotection from **X** to **XI** can be affected under conditions similar like in the conversion of **II** to **XII** and **IV** to **V.**

Finally, compounds of formula I wherein R and R' are not the same, can be synthesized following the route in scheme 6. Compound **VI,** synthesized as in scheme 2, is deprotected under suitable conditions similar like in the conversion of **II** to **XII** and **IV** to **V.** The resulting compound **XV** may then be converted to a compound of formula **XVI** by acylation with the appropriate acid of formula R-C(=O)-OH wherein R has the meanings of R and R' as defined for the compounds of formula I or any subgroup thereof. Compound **XVI** can then be converted under aforementioned Suzuki-Miyaura conditions obtaining compound **X,** which can be further transformed as depicted in scheme 2.

The synthesis procedures as depicted above in schemes 1 to 6 may also be performed using racemic proline derivatives or D-proline derivatives instead of L-proline. Thereby, compounds of formula I with alternative stereochemistry may be obtained.

In a further aspect, the present invention concerns a pharmaceutical composition comprising a therapeutically effective amount of a compound of formula I as specified herein, and a pharmaceutically acceptable carrier. A therapeutically effective amount in this context is an amount sufficient to act in a prophylactic way against HCV infection, to stabilize or to reduce HCV infection, in infected subjects or subjects being at risk of being infected. In still a further aspect, this invention relates to a process of preparing a pharmaceutical composition as specified herein, which comprises intimately mixing a pharmaceutically acceptable carrier with a therapeutically effective amount of a compound of formula I, as specified herein.

Therefore, the compounds of the present invention or any subgroup thereof may be formulated into various pharmaceutical forms for administration purposes. As appropriate compositions there may be cited all compositions usually employed for systemically administering drugs. To prepare the pharmaceutical compositions of this invention, an effective amount of the particular compound, optionally in addition salt form or metal complex, as the active ingredient is combined in intimate admixture with a pharmaceutically acceptable carrier, which carrier may take a wide variety of forms depending on the form of preparation desired for administration. These pharmaceutical compositions are desirable in unitary dosage form suitable, particularly, for administration orally, rectally, percutaneously, or by parenteral injection. For example, in preparing the compositions in oral dosage form, any of the usual pharmaceutical media may be employed such as, for example, water, glycols, oils, alcohols and the like in the case of oral liquid preparations such as suspensions, syrups, elixirs, emulsions and solutions; or solid carriers such as starches, sugars, kaolin, lubricants, binders, disintegrating agents and the like in the case of powders, pills, capsules, and tablets. Because of their ease in administration, tablets and capsules represent the most advantageous oral dosage unit forms, in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. Also included are solid form preparations intended to be converted, shortly before use, to liquid form preparations. In the compositions suitable for percutaneous administration, the carrier optionally comprises a penetration enhancing agent and/or a suitable wetting agent, optionally combined with suitable additives of any nature in minor proportions, which additives do not introduce a significant deleterious effect on the skin. The compounds of the present invention may also be administered via oral inhalation or insufflation in the form of a solution, a suspension or a dry powder using any art-known delivery system.

It is especially advantageous to formulate the aforementioned pharmaceutical compositions in unit dosage form for ease of administration and uniformity of dosage. Unit dosage form as used herein refers to physically discrete units suitable as unitary dosages, each unit containing a predetermined quantity of active ingredient calculated to produce the desired therapeutic effect in association with the required pharmaceutical carrier. Examples of such unit dosage forms are tablets (including scored or coated tablets), capsules, pills, suppositories, powder packets, wafers, injectable solutions or suspensions and the like, and segregated multiples thereof.

The compounds of formula I show activity against HCV and can be used in the treatment and prophylaxis of HCV infection or diseases associated with HCV. The latter include progressive liver fibrosis, inflammation and necrosis leading to cirrhosis, end-stage liver disease, and HCC. A number of the compounds of this invention moreover are believed to be active against mutated strains of HCV. Additionally, compounds of this invention may show a favorable pharmacokinetic profile and have attractive properties in terms of bioavailability, including an acceptable half-life, AUC (area under the curve) and peak values and lacking unfavorable phenomena such as insufficient quick onset and tissue retention.

The *in vitro* antiviral activity against HCV of the compounds of formula I can be tested in a cellular HCV replicon system based on Lohmann et al. (1999) Science 285:110-113, with the further modifications described by Krieger et al. (2001) Journal of Virology 75: 4614-4624 (incorporated herein by reference), which is further exemplified in the examples section. This model, while not a complete infection model for HCV, is widely accepted as the most robust and efficient model of autonomous HCV RNA replication currently available. It will be appreciated that it is important to distinguish between compounds that specifically interfere with HCV functions from those that exert cytotoxic or cytostatic effects in the HCV replicon model, and as a consequence cause a decrease in HCV RNA or linked reporter enzyme concentration. Assays are known in the field for the evaluation of cellular cytotoxicity based for example on the activity of mitochondrial enzymes using fluorogenic redox dyes such as resazurin. Furthermore, cellular counter screens exist for the evaluation of non-selective inhibition of linked reporter gene activity, such as firefly luciferase. Appropriate cell types can be equipped by stable transfection with a luciferase reporter gene whose expression is dependent on a constitutively active gene promoter, and such cells can be used as a counter-screen to eliminate non-selective inhibitors.

Due to their antiviral properties, particularly their anti-HCV properties, the compounds of formula I or subgroups thereof, as specified herein, are useful in the inhibition of HCV replication, in particular in the treatment of warm-blooded animals, in particular humans, infected with HCV, and for the prophylaxis of HCV infections. The present invention furthermore relates to a method of treating a warm-blooded animal, in particular human, infected by HCV, or being at risk of infection by HCV, said method comprising the administration of an anti-HCV effective amount of a compound of formula I, as specified herein.

The compounds of formula I, as specified herein, may therefore be used as a medicine, in particular as an anti HCV medicine. Said use as a medicine or method of treatment comprises the systemic administration to HCV infected subjects or to subjects susceptible to HCV infection of an amount effective to combat the conditions associated with HCV infection.

The present invention also relates to the use of the present compounds in the manufacture of a medicament for the treatment or the prevention of HCV infection. In general it is contemplated that an antiviral effective daily amount would be from about 0.01 to about 50 mg/kg, or about 0.02 to about 30 mg/kg body weight. It may be appropriate to administer the required dose as two, three, four or more sub-doses at appropriate intervals throughout the day. Said sub-doses may be formulated as unit dosage forms, for example, containing about 1 to about 500 mg, or about 1 to about 300 mg, or about 1 to about 100 mg, or about 2 to about 50 mg of active ingredient per unit dosage form.

### Combination therapy

The invention also relates to a combination of a compound of formula I, a pharmaceutically acceptable salt or solvate thereof, and another antiviral compound, in particular another anti-HCV compound. The term "combination" relates to a product containing (a) a compound of formula I, as defined hereinbefore, and (b) another anti-HCV inhibitor, as a combined preparation for simultaneous, separate or sequential use in the treatment of HCV infections.

The combinations of the present invention may be used as medicaments. Accordingly, the present invention relates to the use of a compound of formula (I) or any subgroup thereof as defined above for the manufacture of a medicament useful for inhibiting HCV activity in a mammal infected with HCV viruses, wherein said medicament is used in a combination therapy, said combination therapy in particular comprising a compound of formula (I) and at least one other anti-HCV agent, e.g. IFN-α, pegylated IFN-α, ribavirin, albuferon, taribavirin, nitazoxanide Debio025 or a combination thereof.

Other agents that may be combined with the compounds of the present invention include, for example, nucleoside and non-nucleoside inhibitors of the HCV polymerase, protease inhibitors, helicase inhibitors, NS4B inhibitors and agents that functionally inhibit the internal ribosomal entry site (IRES) and other agents that inhibit HCV cell attachment or virus entry, HCV RNA translation, HCV RNA transcription, replication or HCV maturation, assembly or virus release. Specific compounds in these classes include HCV protease inhibitors such as telaprevir (VX-950), boceprevir (SCH-503034), narlaprevir (SCH-900518), ITMN-191 (R-7227), TMC435350 (TMC435), MK- 7009, BI-201335, BI-2061 (ciluprevir), BMS-650032, ACH-1625, ACH-1095, GS 9256, VX-985, IDX-375 (HCV NS4A protease co-factor inhibitor), VX-500, VX-813, PHX-1766, PHX2054, IDX-136, IDX-316, ABT-450, EP-013420 (and congeners) and VBY-376; the nucleoside HCV polymerase inhibitors useful in the invention include R7128, PSI-7851, PSI 7977, IDX-189,IDX-184, IDX-102, R1479, UNX-08189, PSI-6130, PSI-938 and PSI-879 and various other nucleoside and nucleotide analogs and HCV inhibitors including those derived as 2'-C-methyl modified nucleosides, 4'-aza modified nucleosides, and 7'-deaza modified nucleosides, e.g. 4-amino-1-[5-azido-4-hydroxy-5-hydroxymethyl-3-methyltetrahydrofuran-2-yl]-pyrimidin-2(1H)-one and the bis-2-methylpropanoate ester thereof. Non-nucleoside HCV polymerase inhibitors useful in the invention include HCV-796, HCV-371, VCH-759, VCH-916, VCH-222, ANA-598, MK-3281, ABT-333, ABT-072, PF-00868554, BI-207127, GS-9190, A- 837093, JKT-109, GL-59728, GL-60667, ABT-072, AZD-2795 and 13-cyclohexyl-3-methoxy-17,23-dimethyl-7H-10,6-(methanoiminothioiminoethanooxyethanoiminomethano)indolo[2,1-a][2]benzazepine-14,24-dione 16,16-dioxide.

### EXAMPLES

### Example 1 - synthesis of compounds of formula V

### 1.1 Preparation of intermediate II

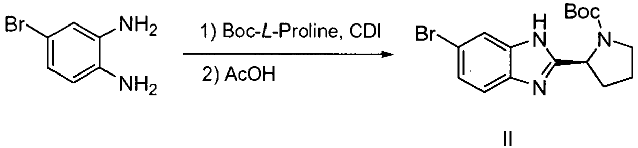

To a solution of Boc-L-Proline (2669 mg, 12.4 mmol) in pyridine/DMF (30 mL, 1/1) was added di(1H-imidazol-1-yl)ketone (2205 mg, 13.6 mmol). The mixture was stirred at 45°C for 2 hours. 4-bromobenzene-1,2-diamine (2319 mg, 12.4 mmol) was added and the mixture was stirred at ambient temperature overnight. The solvent was removed and the residue heated in acetic acid (15 mL) at 100°C for 30 minutes. After concentration of the residue, the mixture was partitioned between ethyl acetate and a saturated sodium bicarbonate solution. The organic phase was separated and washed with water. After drying over Na₂SO₄, the mixture was filtered and the filtrate was concentrated in vacuo. The obtained residue was purified by flash chromatography using DCM/EtOAc 90/10 to 50/50, resulting in compound II (3.146 g, 69 %).

### 1.2 Preparation of intermediate III

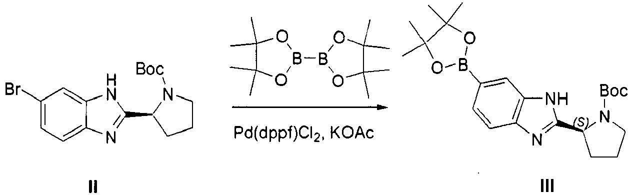

To a mixture of II (200 g, 546 mmol), potassium acetate (160.8 g, 1.64 mol) and 4,4,4',4',5,5,5',5'-octamethyl-2,2'-bi(1,3,2-dioxaborolane) (416 g, 1.64 mol) in DMF (3L) was added Pd(dppf)Cl₂ (20 g) under nitrogen gas. The reaction mixture was stirred at 85 °C for 15 hours. The mixture was diluted with ethyl acetate, washed with water and brine, dried over magnesium sulfate, the solids removed by filtration, and the solvents of the filtrate were removed under reduced pressure. The residue was purified by silica column chromatography (petroleum ether : ethyl acetate 10:1 to 2:1) to afford 125 g of III as a white solid (contains 15% of boronic acid).

### 1.3 Preparation of intermediate XVII

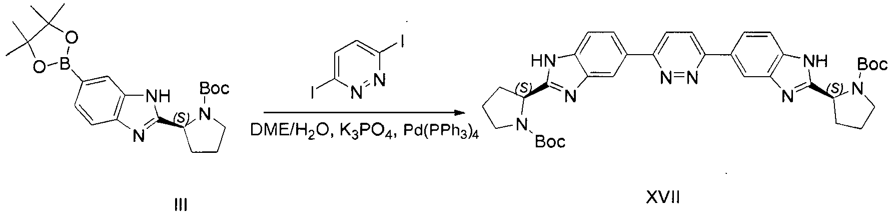

To 3,6-diiodopyridazine (1156 mg, 3.48 mmol), tetrakis(triphenylphosphine)palladium, 281.8 mg, 0.244 mmol), K₃PO₄ (1479 mg, 6.97 mmol) and compound III (3.6 g, 8.71 mmol), DME (30 mL) and H₂O (10 mL) were added. The vigorously stirred mixture was warmed to 90°C under a nitrogen atmosphere and stirred at this temperature overnight.
Next, DCM (20 mL) was added followed by aqueous Na₂CO₃ (2M, 1.5 mL) containing concentrated aqueous ammonia (0.3 mL). The organic layer was separated and the water layer extracted with DCM. The combined organic layers were dried over Na₂SO₄ and after fltration, concentrated to dryness under reduced pressure to afford a brown residue. This residue was purified by column chromatography with DCM to DCM/MeOH (7N NH3) 95/5 as eluent, resulting in compound XVII (680 mg, 30 %) as a foam.

### 1.4 Preparation of intermediate XVIII

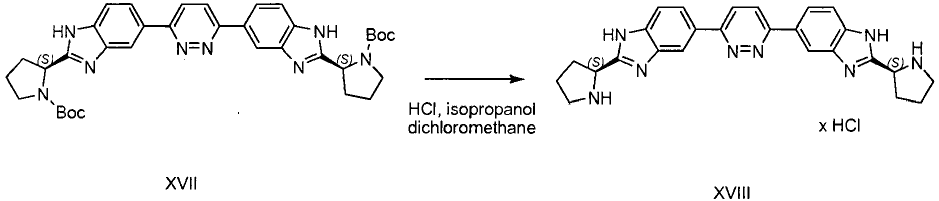

To a solution of XVII (730 mg, 1.12 mmol) in isopropanol (5mL) was added HCl (5-6 M in isopropanol, 5 mL). The mixture was stirred at room temperature. After 4 hours, more HCl (5-6M inisopropanol, 10 mL) was added and the mixture was further stirred at room temperature overnight. The solvent was evaporated, the obtained solid was dried in vacuo and used as such in the next step.

### Example 2 - synthesis of comnounds of formula I

### 2.1. Preparation of compound nr. 1

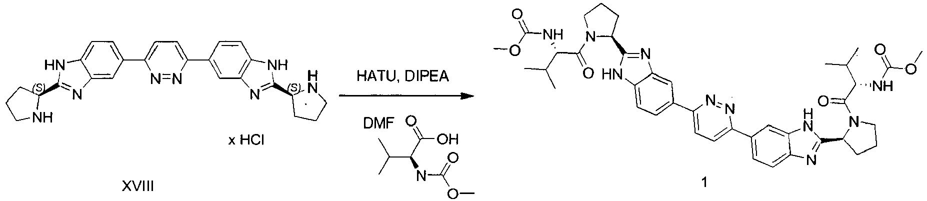

To a solution of XVIII (250 mg, ∼0.40 mmol) in dry DMF (5 mL) was added DIPEA (0.734 mL, 4.439 mmol), HATU (527 mg, 1.4 mmol) and acid (389 mg, 2.22 mmol). The mixture was stirred for 2 hours at room temperature. DCM was added and the mixture was washed with saturated NaHCO₃ (2 x 20 ml). The organic phase was dried on MgSO₄ and after filtration, concentrated in vacuo. Purification was performed by silica gel chromatography (0-8% MeOH in DCM), resulting in compound 1 as a solid (200 mg, 0.261 mmol). Rt: 4.99 min. m/z=: 765.4 (M+1)+ Exact mass: 764.4

¹H NMR (400 MHz, DMSO-d6) δ ppm 12.29 - 12.65 (2 H, m) 8.21 - 8.73 (4 H, m) 7.89 - 8.20 (2 H, m) 7.55 - 7.75 (2 H, m) 6.78 - 7.50 (2 H, m) 5.16 - 5.49 (2 H, m) 4.01 - 4.15 (2 H, m) 3.78 - 3.95 (4 H, m) 3.55 (6 H, s) 2.17 - 2.37 (4 H, m) 1.84 - 2.16 (6 H, m) 0.77 - 0.96 (12 H, m)

### 2.2 preparation of compounds 2 to 9

Compounds 2 to 9 as listed in table 1 were synthesized using the procedure for compound 1 as described in example 3.1 using the appropriate carboxylic acid of formula R/R'-C(=O)=OH and the appropriate bis-benzimidazole scaffolds.

All compounds were characterized by LC/MS.

Liquid Chromatography: Waters Alliance 2695, UV detector:Waters 996 PDA, range:210-400 nm; Mass detector: Waters ZQ, ion source: ES+, ES- Column used: SunFire C18 3.51µ 4.6x100 mm mobile phase A: 10mM NH₄OOCH+ 0.1% HCOOH in H₂O; mobile phase B: CH₃OH; column temp.: 50°C; flow: 1.5mL/min

Gradient time(min) [%A/%B]0 [65/35] to 7[5/95] to 9.6[5/95] to 9.8[65/35] to 12 [65/35]

**Table 1 - compounds of formula I**

| | | | | | | |
|---|---|---|---|---|---|---|
| 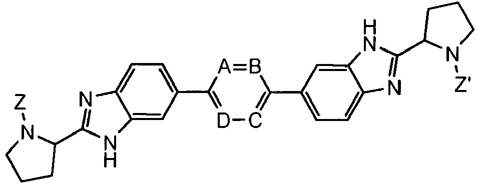 | | | | | | |

| **Co. nr.** | **Z(* denotes point of attachment)** | **Z' (* denotes point of attachment)** | 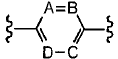 | **Exact Mass** | **Observed Mass (M+H)** | **Rt (Minutes)** |
|---|---|---|---|---|---|---|
| 1 | 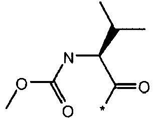 | 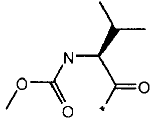 | 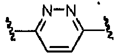 | 764.4 | 765.4 | 4.99 |
| 2 | 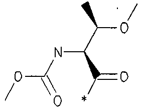 | 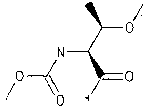 | 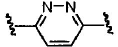 | 796.4 | 797.4 | 4.40 |
| 3 | 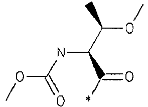 | 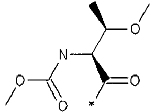 | 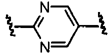 | 796.4 | 797.5 | 4.86 |
| 4 | 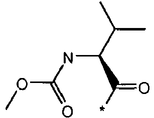 | 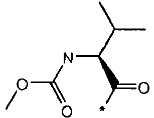 | 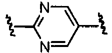 | 764.4 | 765.4 | 5.43 |
| 5 | 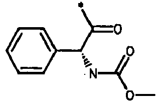 | 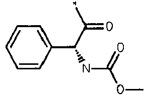 | 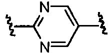 | 832.3 | 833.4 | 5.67 |
| 6 | 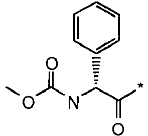 | 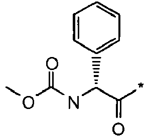 | 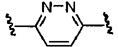 | 832.3 | 833.4 | 5.31 |
| 7 | 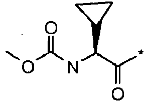 | 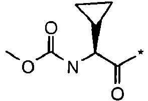 | 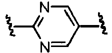 | 760.3 | 761.4 | 4.97 |
| 8 | 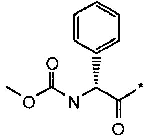 | 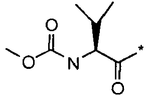 | 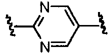 | 798.4 | 799.4 | 5.58 |
| 9 | 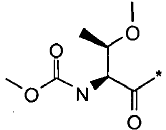 | 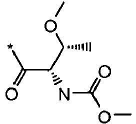 | 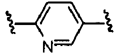 | 795.4 | 796.5 | 4.72 |

Compound **2:** ¹H NMR (400 MHz, DMSO-*d*₆) δ ppm 12.29 - 12.65 (2 H, m) 8.23 - 8.42 (4 H, m) 8.10 ( H, d, *J*=8.4 Hz) 7.98 (1 H, d, *J*=8.4 Hz) 7.67 (1 H, d, *J*=8.4 Hz) 7.61 (1 H, d, *J*=8.4 Hz) 6.74 - 7.28 (2 H, m) 5.15 - 5.65 (2 H, m) 4.32 (2 H, t, *J*=7.3 Hz) 3.83 - 3.94 (4 H, m) 3.43 - 3.62 (8 H, m) 3.20 (6 H, s) 2.15 - 2.38 (4 H, m) 1.81 - 2.16 (4 H, m) 1.03 - 1.15 (6 H, m)
Compound **4**: ¹H NMR (400 MHz, DMSO-*d*₆) δ ppm 12.23 - 12.74 (2 H, m) 9.19-9.22 (2 H, m) 8.52 - 8.61(1 H, m) 8.26 - 8.37 (1 H, m) 7.85 - 8.06 (1 H, m) 7.53 - 7.70 (3 H, m) 7.27 - 7.37(2 H, m) 5.17 - 5.25 (2 H, m) 3.99 - 4.15 (2H, m) 3.76 - 3.94 (4H, m) 3.55 (6 H, s) 2.18 - 2.31 (4 H, m) 1.85-2.14 (6 H, m) 0.75-0.93 (12 H, m)

### Example 3 - anti-HCV activity of compounds of formula I

### Replicon assay

The compounds of formula (1) were examined for inhibitory activity in the HCV replicon. This cellular assay is based on a bicistronic expression construct, as described by Lohmann et al. (1999) Science vol. 285 pp. 110-113 with modifications described by Krieger et al. (2001) Journal of Virology 75: 4614-4624, in a multi-target screening strategy.

In essence, the method was as follows:
The assay utilized the stably transfected cell line Huh-7 luc/neo (hereafter referred to as Huh-Luc). This cell line harbors an RNA encoding a bicistronic expression construct comprising the wild type NS3-NS5B regions of HCV type 1b translated from an Internal Ribosome Entry Site (IRES) from encephalomyocarditis virus (EMCV), preceded by a reporter portion (FfL-luciferase), and a selectable marker portion (neoR, neomycine phosphotransferase). The construct is flanked by 5' and 3' NTRs (non-translated regions) from HCV type 1b. Continued culture of the replicon cells in the presence of G418 (neoR) is dependent on the replication of the HCV RNA. The stably transfected replicon cells that express HCV RNA, which replicates autonomously and to high levels, encoding inter alia luciferase, were used for screening the antiviral compounds.

The replicon cells were plated in 384 well plates in the presence of the test and control compounds which were added in various concentrations. Following an incubation of three days, HCV replication was measured by assaying luciferase activity (using standard luciferase assay substrates and reagents and a Perkin Elmer ViewLux^{™} ultraHTS microplate imager). Replicon cells in the control cultures have high luciferase expression in the absence of any inhibitor. The inhibitory activity of the compound on luciferase activity was monitored on the Huh-Luc cells, enabling a dose-response curve for each test compound. EC₅₀ values were then calculated, which represent the amount of compound required to decrease the level of detected luciferase activity by 50%, or more specifically, to reduce the ability of the genetically linked HCV replicon RNA to replicate.

### Results

**Table 1 shows the replicon results obtained for compounds of the examples given above.**

| **STRUCTURE** | **Compound no.** | **HCV-REP-HUH-LUC_EC₅₀ (µM)** |
|---|---|---|
| 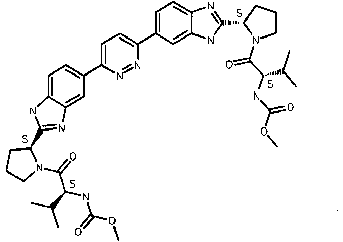 | 1 | 0.00013 |
| 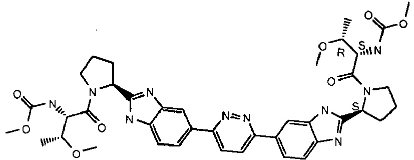 | 2 | |
| 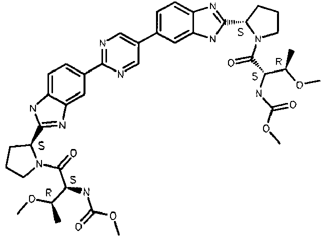 | 3 | 0.00013 |
| 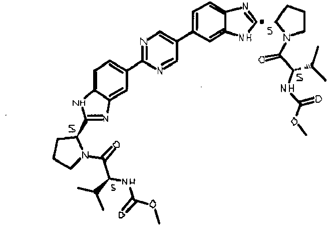 | 4 | 0.00003 |
| 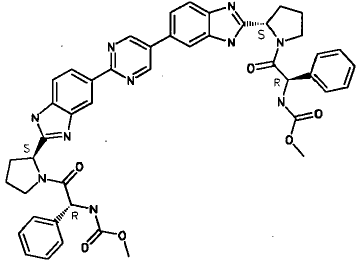 | 5 | <0.000015 |
| 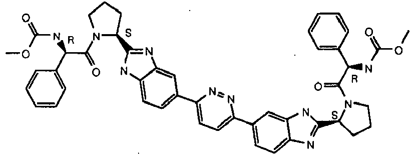 | 6 | 0.000045 |
| 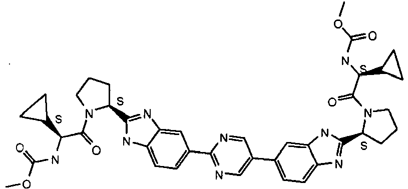 | 7 | 0.000059 |
| 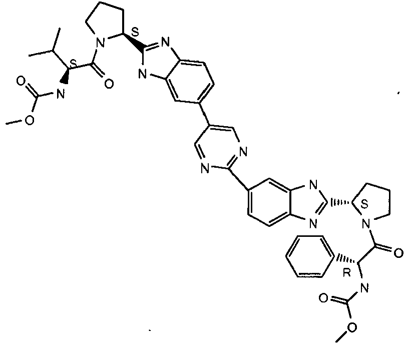 | 8 | |
| 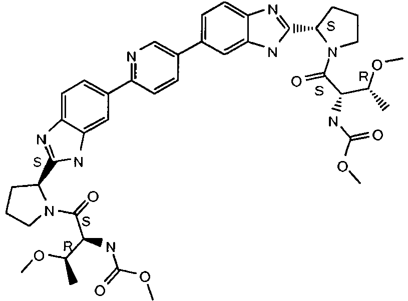 | 9 | 0.00031 |

## Claims

1. A compound of formula **I** 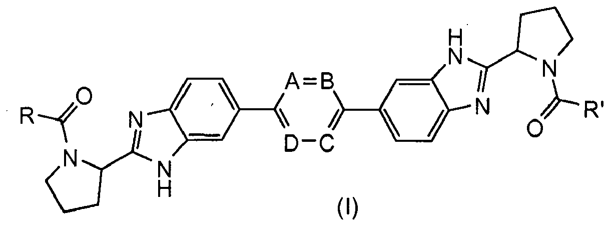 or a stereoisomer thereof, wherein:
A, B, C and D are independently -CH= or -N=, provided that one or two of A, B, C and D is -N= and the remainder is -CH=;
R and R' are independently selected from-CR₁R₂R₃, aryl optionally substituted with 1 or 2 substituents selected from halo and methyl, and heteroC₄₋₇cycloalkyl, wherein
R₁ is selected from the group consisting of C₁₋₄alkyl optionally substituted with methoxy or dimethylamino; phenyl optionally substituted with 1, 2 or 3 substituents independently selected from halo, C₁₋₄alkoxy, trifluoromethoxy or 2 substituents on adjacent ring atoms form a 1,3-dioxolane group; benzyl optionally substituted with halo or methoxy; C₃₋₆cycloalkyl; heteroaryl; heteroC₄₋₇cyclo-alkyl; and heteroarylmethyl;
R₂ is selected from the group consisting of hydrogen, hydroxyl, amino, mono- and di-C₁₋₄alkylamino, C₁₋₄alkylcarbonylamino, C₁₋₄alkyloxycarbonylamino, C₁₋₄alkylaminocarbonylamino, piperidin-1-yl and imidazol-1-yl; and
R₃ is hydrogen, or R₁ and R₃ together form a cyclopropyl group; or R₂ and R₃ together form oxo;
or a pharmaceutically acceptable salt or a solvate thereof, provided that 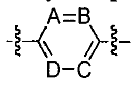 is other than pyrazine or pyridine when R and R' both are (S)-1-methoxycarbonylamino-2-methyl-propan-1-yl; and that R² is other than methoxycarbonylamino when R¹ is phenyl and R³ is hydrogen.

2. The compound according to claim 1 wherein R and R' are independently selected from -CR₁R₂R₃.

3. The compound according to claim 1 wherein R and R' are the same.

4. The compound according to claim 1 wherein R₂ is C₁₋₄alkylcarbonylamino or C₁₋₄alkyloxycarbonylamino.

5. The compound according to claim 1 wherein R₁ is selected from branched C₃₋₄alkyl; phenyl optionally substituted with 1 substituent selected from halo and methyl; and heteroaryl.

6. The compound according to claim 1 wherein R₁ is selected from C₁₋₄alkyl optionally substituted with methoxy; phenyl optionally substituted with halo, and C₃₋₆cycloalkyl.

7. The compound according to claim 1 wherein the compound is of formula Ia.

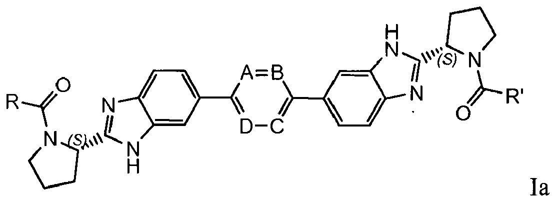

8. The compound according to claim 1 wherein 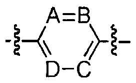 is pyrimidine or pyridazine.

9. A pharmaceutical composition comprising a compound as defined in any of claims 1 to 8, and a pharmaceutically acceptable carrier.

10. A compound as defined in any of claims 1 to 8 or a pharmaceutical composition as defined in claim 9, for use in the prevention or treatment of an HCV infection in a mammal.

## Patentansprüche

1. Verbindung der Formel I 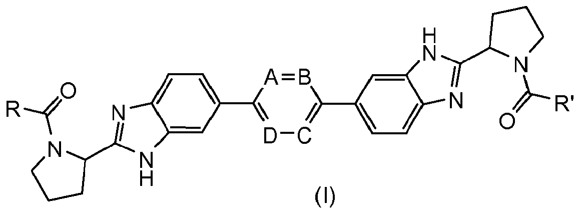 oder ein Stereoisomer davon, wobei:
A, B, C und D unabhängig voneinander für -CH= oder -N= stehen, mit der Maßgabe, dass eine oder zwei der Variablen A, B, C und D für -N= stehen und der Rest für -CH= steht;
R und R' unabhängig voneinander aus -CR₁R₂R₃, Aryl, das gegebenenfalls durch 1 oder 2 aus Halogen und Methyl ausgewählte Substituenten substituiert ist, und Hetero-C₄₋₇-cycloalkyl ausgewählt sind, wobei
R₁ aus der Gruppe bestehend aus C₁₋₄-Alkyl, das gegebenenfalls durch Methoxy oder Dimethylamino substituiert ist; Phenyl, das gegebenenfalls durch 1, 2 oder 3 unabhängig aus Halogen, C₁₋₄-Alkoxy, Trifluormethoxy ausgewählte Substituenten substituiert ist, wobei 2 Substituenten an benachbarten Ringatomen auch eine 1,3-Dioxolangruppe bilden können; Benzyl, das gegebenenfalls durch Halogen oder Methoxy substituiert ist; C₃₋₆-Cycloalkyl; Heteroaryl; Hetero-C₄₋₇-cycloalkyl und Heteroarylmethyl ausgewählt ist;
R₂ aus der Gruppe bestehend aus Wasserstoff, Hydroxyl, Amino, Mono- und Di-C₁₋₄-alkylamino, C₁₋₄-Alkylcarbonylamino, C₁₋₄-Alkyloxycarbonyl-amino, C₁₋₄-Alkylaminocarbonylamino, Piperidin-1-yl und Imidazol-1-yl ausgewählt ist; und
R₃ für Wasserstoff steht oder R₁ und R₃ zusammen eine Cyclopropylgruppe bilden; oder R₂ und R₃ zusammen Oxo bilden;
oder ein pharmazeutisch unbedenkliches Salz oder Solvat davon,
mit den Maßgaben, dass 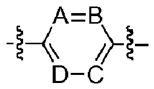 von Pyrazin oder Pyridin verschieden ist, wenn R und R' beide für (S)-1-Methoxycarbonylamino-2-methylpropan-1-yl stehen; und dass R² von Methoxycarbonylamino verschieden ist, wenn R¹ für Phenyl steht und R³ für Wasserstoff steht.

2. Verbindung nach Anspruch 1, wobei R und R' unabhängig voneinander aus -CR₁R₂R₃ ausgewählt sind.

3. Verbindung nach Anspruch 1, wobei R und R' gleich sind.

4. Verbindung nach Anspruch 1, wobei R₂ für C₁₋₄-Alkylcarbonylamino oder C₁₋₄-Alkyloxycarbonylamino steht.

5. Verbindung nach Anspruch 1, wobei R₁ aus verzweigtem C₃₋₄-Alkyl; Phenyl, das gegebenenfalls durch 1 aus Halogen und Methyl ausgewählten Substituenten substituiert ist; und Heteroaryl ausgewählt ist.

6. Verbindung nach Anspruch 1, wobei R₁ aus C₁₋₄-Alkyl, das gegebenenfalls durch Methoxy substituiert ist; Phenyl, das gegebenenfalls durch Halogen substituiert ist, und C₃₋₆-Cycloalkyl ausgewählt ist.

7. Verbindung nach Anspruch 1 mit der Formel Ia.

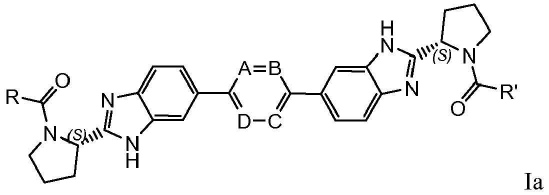

8. Verbindung nach Anspruch 1, wobei 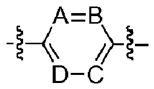 für Pyrimidin oder Pyridazin steht.

9. Pharmazeutische Zusammensetzung, umfassend eine Verbindung gemäß einem der Ansprüche 1 bis 8 und einen pharmazeutisch unbedenklichen Träger.

10. Verbindung gemäß einem der Ansprüche 1 bis 8 oder pharmazeutische Zusammensetzung gemäß Anspruch 9 zur Verwendung bei der Prävention oder Behandlung einer HCV-Infektion bei einem Säugetier.

## Revendications

1. Composé de formule I 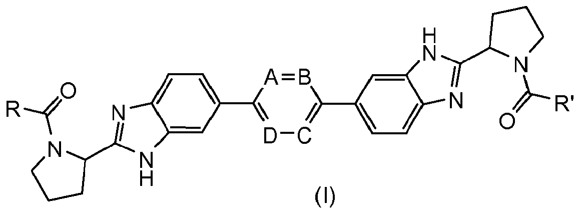 ou un stéréoisomère de celui-ci, dans lequel
A, B, C et D sont indépendamment -CH= ou -N=, à condition que un ou deux parmi A, B, C et D soient -N= et que le reste soit -CH= ;
R et R' sont choisis indépendamment parmi -CR₁R₂R₃, aryle éventuellement substitué par 1 ou 2 substituants choisis parmi halogéno et méthyle, et hétéroC₄₋₇-cycloalkyle, où
R₁ est choisi dans le groupe constitué par C₁₋₄alkyle éventuellement substitué par méthoxy ou diméthylamino ; phényle éventuellement substitué par 1, 2 ou 3 substituants choisis indépendamment parmi halogéno, C₁₋₄-alcoxy, trifluorométhoxy ou 2 substituants sur des atomes de cycle adjacents forment un groupement 1,3-dioxolane ; benzyle éventuellement substitué par halogéno ou méthoxy ; C₃₋₆cycloalkyle ; hétéroaryle ; hétéroC₄₋₇cycloalkyle ; et hétéroarylméthyle ;
R₂ est choisi dans le groupe constitué par hydrogène, hydroxyle, amino, mono- et di-C₁₋₄alkylamino, C₁₋₄-alkylcarbonylamino, C₁₋₄alkyloxycarbonylamino, C₁₋₄-alkylaminocarbonylamino, pipéridin-1-yle et imidazol-1-yle ; et
R₃ est hydrogène, ou R₁ et R₃ forment ensemble un groupement cyclopropyle ; ou R₂ et R₃ forment ensemble oxo ;
ou un sel pharmaceutiquement acceptable ou un solvat de celui-ci,
à condition que 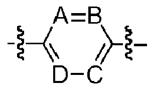 soit autre que pyrazine ou pyridine lorsque R et R' sont tous deux (S)-1-méthoxycarbonylamino-2-méthylpropan-1-yle ; et que R₂ soit autre que méthoxycarbonylamino lorsque R₁ est phényle et R₃ est hydrogène.

2. Composé selon la revendication 1, dans lequel R et R' sont choisis indépendamment parmi -CR₁R₂R₃.

3. Composé selon la revendication 1, dans lequel R et R' sont identiques.

4. Composé selon la revendication 1, dans lequel R₂ est C₁₋₄alkylcarbonylamino ou C₁₋₄alkyloxycarbonylamino.

5. Composé selon la revendication 1, dans lequel R₁ est choisi parmi C₃₋₄alkyle ramifié ; phényle éventuellement substitué par 1 substituant choisi parmi halogéno et méthyle ; et hétéroaryle.

6. Composé selon la revendication 1, dans lequel R₁ est choisi parmi C₁₋₄alkyle éventuellement substitué par méthoxy ; phényle éventuellement substitué par halogéno ; et C₃₋₆cycloalkyle.

7. Composé selon la revendication 1, **caractérisé en ce que** le composé répond à la formule la

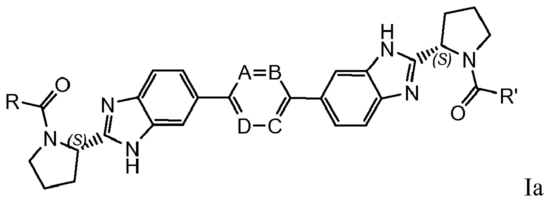

8. Composé selon la revendication 1, dans lequel 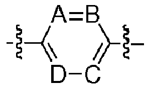 est pyrimidine ou pyridazine.

9. Composition pharmaceutique comprenant un composé tel que défini selon l'une quelconque des revendications 1 à 8, et un véhicule pharmaceutiquement acceptable.

10. Composé tel que défini selon l'une quelconque des revendications 1 à 8, ou une composition pharmaceutique telle que définie selon la revendication 9, pour une utilisation dans la prévention ou le traitement d'une infection par le VHC chez un mammifère.
